# EUROPEAN PATENT SPECIFICATION

(11) **EP 1 389 478 B1**
(45) Date of publication and mention of the grant of the patent: **19.04.2006**
(21) Application number: 03076335.3
(22) Date of filing: 06.05.2003
(51) Int. Cl.: A61M 25/01, A61B 18/02

(54) **Catheter having articulation system made from two different materials**
Katheter mit Lenkeinrichtung bestehend aus zwei unterschiedlichen Materialien
Cathéter avec dispositif d'orientation fabriqué de deux matériaux différents

(30) Priority: 16.08.2002 US 223077
(43) Date of publication of application: 18.02.2004
(73) Proprietor: Cryocor, Inc., San Diego, California 92121 (US)
(72) Inventor: Lentz, David J., La Jolla, California 92037 (US); Salinas, Alvin B., San Marcos, California 92069 (US)
(74) Representative: Shortt, Peter Bernard

(56) References cited:
- EP-A- 1 046 406
- WO-A-01/78825
- WO-A-02/30310
- WO-A-94/11057
- US-A1- 2001 049 491
- US-B1- 6 270 476

## Description

### FIELD OF THE INVENTION

The present invention pertains generally to medical catheters. More particularly, the present invention pertains to articulation segments for catheters that increase catheter steerability and allow the distal portion of the catheter to be configured into a preselected shape at a target site in a patient's vasculature. The present invention is particularly, but not exclusively, useful as an articulation system for a cardiac cryoablation catheter.

### BACKGROUND OF THE INVENTION

Steerability, among several attributes, is an important consideration in the manufacture and operation of an invasive catheter. In particular, when the operation of a catheter requires that it be advanced through portions of a patient's vasculature, the ability to steer the catheter along tortuous paths, and into selected branches of the vasculature, is of crucial importance. Further, in addition to having good steering properties, it may also be important to reconfigure the distal end of the catheter into a desirable shape once the catheter has been advanced to a position near the target tissue. In either case, the steering and configuring of an invasive catheter requires that the distal tip of the catheter be articulated in a safe, predictable and controllable manner.

One particular application in which a highly articulatable catheter is beneficial is in the treatment of atrial fibrillation. Atrial fibrillation is an irregular heart rhythm that adversely affects approximately 2.5 million people in the U.S. It is believed that at least one-third of all atrial fibrillation originates near the ostium of the pulmonary veins, and that the optimal treatment technique is to ablate conductive pathways (i.e. create conduction blocks) associated with focal atrial fibrillation. In greater detail, creation of circumferential and linear lesions via ablation near the ostia of the pulmonary veins has been shown to be an effective treatment for atrial fibrillation. However, accessing the pulmonary veins near the ostia using a catheter, requires a catheter that is highly articulatable.

Several devices have been previously suggested for the purpose of steering a catheter through the vasculature of a patient. In the earlier mechanisms, such as the one disclosed in U.S. Patent No. 1,060,665, that issued to Bell on May 6, 1913, for an invention entitled "Catheter", the steerability of the catheter was provided for by using a pre-bent stiffening member in the catheter's distal end. Subsequently, more complex devices have relied on a pull-wire to deflect the catheter tip. In general, these mechanisms have variously included concentric or eccentric pull-wires that generate an eccentrically applied force on the tip of the catheter. For example, U.S. Patent No. 4,456,017, which issued to Miles for an invention entitled "Coil Spring Guide with Deflectable Tip" incorporates a concentric core wire for this purpose. On the other hand, U.S. Patent No. 4,586,923, which issued to Gould et al., uses an eccentric wire for the same purpose. Further, devices have also been proposed which will bias the deflection of a catheter tip in a predetermined plane. An example of such a device is disclosed in U.S. Patent No. 4,886,067, which issued to Palermo. In the Palermo patent, such a bias is established by flattening the core wire.

Heretofore, as indicated by the examples given above, the steerability of a catheter tip has been primarily engineered by determining the direction in which a deflecting force should be applied to the tip. Accordingly, these earlier devices did not specifically incorporate structural aspects into the construction of a catheter's distal portion with a view toward using this construction as a functional aspect for tip deflection. Such a consideration, however, becomes more significant when, in addition to steerability, the configurability of a catheter in the vasculature of a patient is an important consideration.

In accordance with well known engineering applications, structures will predictably bend according to the shape of the structure and according to particular properties of the material, such as its flexural modulus. Importantly. the shape and flexural modulus of a structure can be used to predict how the structure will bend in response to a given force. Further, for structures having both relatively stiff components and relatively flexible components, the bending of the overall structure will generally be dictated by the shape and stiffness of the stiff component.

WO 01/78825 describes a steerable device for introducing diagnostic and therapeutic apparatus into the body. The device includes an elongate body having a lumen extending therethrough and a steering wire associated with the distal portion of the elongate body. Elongate body distal portions may be constructed to include a relatively soft, flexible member and a relatively hard, less flexible member. US 6,270,476 describes a catheter for obtaining tissue, for example to treat a cardial arrhythmia, which includes a handle, a shapeable shaft and a distal ablation segment. US Application 2001/0049491 describes a process for producing a steerable sheath catheter, having a distal end, a proximal end, an outer jacket, a pull wire and a central lumen. EP 1,046,406 describes an actuator for use with a catheter with independent proximal and distal control.

In light of the above, it is an object of the present invention to provide a device for steering a cardiac cryoablation catheter through the vasculature (including areas in and around the heart) of a patient that can be both steered and configured, as desired, while the catheter is in the vasculature of a patient. Another object of the present invention is to provide an articulation segment for a cardiac cryoablation catheter that bends relatively easily but yet has good axial stiffness and torqueability. It is yet another object of the present invention to provide an articulation segment for a cardiac cryoablation catheter that predictably bends in a pre-determined bend plane in response to the movement of a control wire. Still another object of the present invention is to provide an articulation segment for a cardiac cryoablation catheter that is relatively easy to manufacture, is simple to use, and is comparatively cost effective.

### SUMMARY OF THE PREFERRED EMBODIMENTS

The present invention is directed to a system for articulating a catheter in the vasculature of a patient, said system comprising: a co-extruded articulation segment having an elongated, cylindrically shaped wall extending from a distal end to a proximal end and surrounding a central lumen therebetween, said wall defining a longitudinal axis and formed with a first section made of a first material having flexural modulus, M₁, and a second section made of a second material having flexural modulus, M₂, wherein M₂ is larger than M₁ (M₂ > M₁), said second section of said articulation segment being elongated in a direction parallel to said longitudinal axis and subtending a substantially constant arc angle, α, of approximately forty-five degrees from said distal end to said proximal end; a tip member affixed to the distal end of said articulation segment; a control wire having a portion disposed in said central lumen of said articulation segment, said control wire having a first end and a second end with said first end attached to said tip member at an attachment point, said attachment point being distanced radially from said longitudinal axis; and a means engaged with the second end of said control wire for axially moving said control wire to selectively bend said articulation segment and deflect said tip member through an arc, θ, in a plane to articulate the catheter.

The invention is also directed to a method for manufacturing a catheter having an articulation system, said method comprising the steps of: co-extruding an elongated, cylindrically shaped wall having a first section made of a first material having flexural modulus, M₁, and a second section made of a second material having flexural modulus, M₂, wherein M₂ is larger than M₁ (M₂ > M₁) and wherein said second section subtends a substantially constant arc angle, α, of approximately forty-five degrees from a distal end to a proximal end; affixing a tip member to said wall; connecting a catheter tube to said wall; attaching a control wire to said tip member at an attachment point with the control wire extending proximally through said catheter tube; and engaging said control wire with a control means for pulling said control wire in a proximal direction to bend said wall and deflect said tip member through an arc, θ, in a plane defined by said second section of said wall and said attachment point.

A catheter having an articulation system for steering the catheter through the vasculature of a patient includes an articulation segment having a cylindrically shaped wall that is connected to the distal end of a catheter tube. The cylindrically shaped wall defines a longitudinal axis and surrounds a central lumen that extends between the proximal and distal ends of the articulation segment. A tip member is affixed to the distal end of the articulation segment.

For the present invention, the wall of the articulation segment is formed with a first section made of a first material having flexural modulus, M₁, and a second section made of a second material having flexural modulus, M₂, with M₂ being larger than M₁, (M₂ > M₁). Relative to the longitudinal axis of the articulation segment, the first and second sections are, in general, diametrically opposed to each other. A preferred first material for the first section is a polyether block amide (PEBA) such as a PEBAX® and a preferred second material for the second section is a polyamide such as Nylon 12.

One end of a control wire is attached to the tip member, while the control wire itself extends from the tip member, through the lumen of the articulation segment and through the catheter tube. As intended for the present invention, the control wire is connected to the tip member at an attachment point that lies at a radial distance from the longitudinal axis of the articulation segment. In a particular embodiment, the attachment point is positioned to interpose the longitudinal axis of the articulation segment between the attachment point and the second section.

In addition, the system includes a mechanism that is engaged with the control wire at the proximal end of the catheter tube for axially pulling on the control wire. In response to a pulling of the control wire in a proximal direction, the flexible articulation segment allows the tip member to be deflected for the purpose of steering or configuring the catheter in the vasculature of a patient. Also, with this cooperation of structure, the position and shape of the high modulus material (i.e. the second section) can be arranged relative to the position of the attachment point, as indicated above, to cause the tip member to deflect in a pre-selected plane in response to axial movements of the control wire.

In one particular application, the articulation system is used as part of a cardiac cryoablation catheter. In this application, the tip member is made of a material having a relatively high thermal conductivity. Additionally, a refrigerant source is provided to supply a fluid that can be cooled to a temperature of approximately minus eighty degrees Celsius. A transfer tube extends from the refrigerant source and passes through the catheter tube and through the lumen of the articulation segment, interconnecting the refrigerant source in fluid communication with the tip member. With this connection, the fluid can be circulated through the tip member during a cardiac cryoablation procedure.

In a particular embodiment of the articulation segment, the second section (i.e. the high modulus material, M₂) is embedded in the wall of the articulation segment. More specifically, the cylindrically shaped wall extends from a cylindrical inner surface to a cylindrical outer surface. At and near the inner surface, the wall is made of a low modulus material, M₁. Also, at and near the outer surface, the wall is made of a low modulus material, M₁. Between the two wall surfaces, a section of high modulus material, M₂ is embedded in the wall. The section of high modulus material, M₂ preferably extends from the distal end to the proximal end of the articulation segment, and extends around the longitudinal axis through an azimuthal angle of approximately forty-five degrees (45°). Also in this embodiment, an open lumen is formed in the wall between the inner and outer surfaces of the wall. Preferably, the open lumen is positioned approximately one-hundred and eighty degrees (180°) around the longitudinal axis from the high modulus material, M₂ (i.e. the open lumen is diametrically opposed to the second section of high modulus material M₂).

In another particular embodiment of the articulation segment, the second section (i.e. the high modulus material, M₂) extends from the inner surface of the wall to the outer surface of the wall, and forms part of these surfaces. Similar to the embodiment described above, the section of high modulus material, M₂ preferably extends from the distal end to the proximal end of the articulation segment, and extends around the longitudinal axis through an azimuthal angle of approximately forty-five degrees (45°). The remainder of the wall is made of the low modulus material, M₁. As a modification of either embodiment disclosed above, a metallic coil or braid can be embedded in the wall, between the inner and outer surfaces to axially stiffen the articulation segment.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a perspective view of a catheter incorporating an articulation system in accordance with the present invention, as it is being advanced into the vasculature of a patient for an invasive procedure;
Fig. 2 is a segmented, perspective view of a cryoablation catheter having the articulation system of the present invention;
Fig. 3 is a sectional view of the distal end portion of the catheter shown in Fig. 2 as seen along the line 3-3 in Fig. 2;
Fig. 4 is a perspective view of a test fixture for measuring flexural modulus;
Fig. 5 is a sectional view of an exemplary articulation segment as seen along line 4-4 in Fig. 2;
Fig. 6 is a perspective view of another embodiment of an articulation segment, with portions removed for clarity, in which the section of high modulus material extends from the inner wall surface to the outer wall surface of the articulation segment;
Fig. 7 is a sectional view of the articulation segment shown in Fig. 6 as seen along line 7-7 in Fig. 6;
Fig. 8 is a perspective view of another embodiment of an articulation segment, with portions removed for clarity, having a metallic braid embedded in the articulation segment wall; and
Fig. 9 is a side plan view of the distal end portion of the catheter shown in Fig. 2, shown after deflection of the distal tip.

### DESCRIPTION OF THE PREFERRED EMBODIMENT

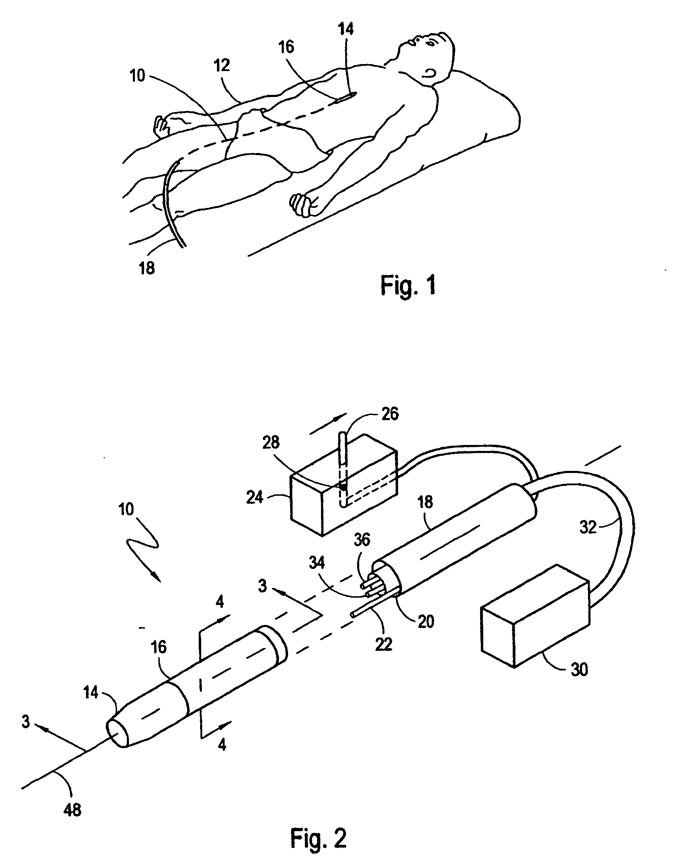

Referring initially to Fig. 1, a catheter for cryoablating internal target tissue in accordance with the present invention is shown and is designated 10. In Fig. 1, the catheter 10 is shown as it is being positioned in the vasculature of a patient 12. The term "vasculature" including derivatives thereof, is herein intended to mean any cavity or lumen within the body which is defined at least in part by a tissue wall, to specifically include the cardiac chambers, arterial vessels and the venous vessels. As further shown in Fig. 1, the catheter 10 includes a tip member 14 that is located at the distal end of the catheter 10 and an articulation segment 16 that is attached proximal to the tip member 14. Still further, a catheter tube 18 is attached to the articulation segment 16. In use, the catheter 10 is manipulated until the tip member 14 is positioned adjacent the target tissue. With the tip member 14 positioned adjacent the target tissue, a low temperature refrigerant is then introduced into the tip member 14, causing heat to flow from the target tissue, through the tip member 14 and into the refrigerant. This results in the cryoablation of the target tissue.

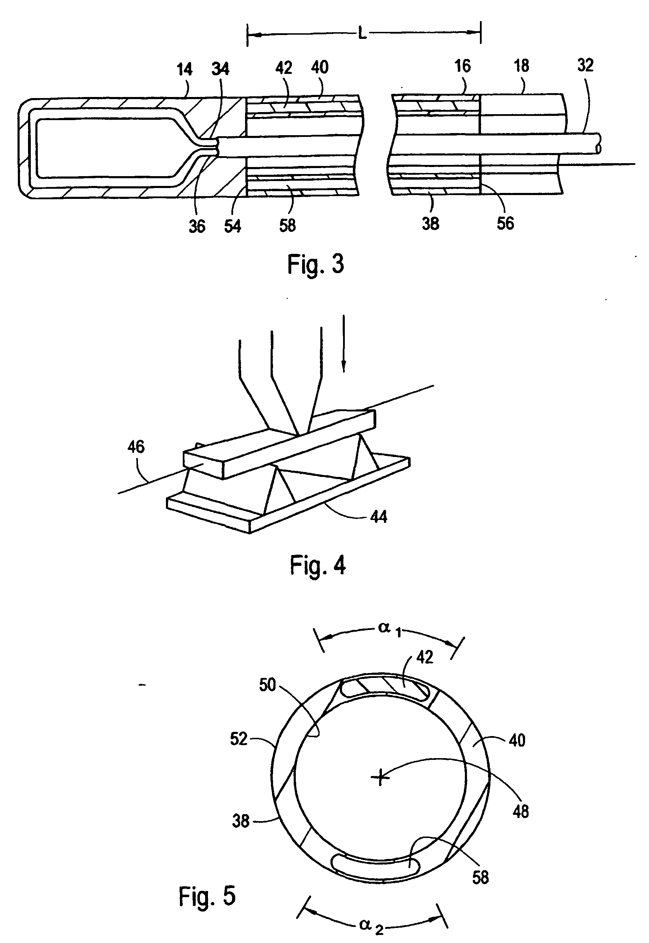

Referring now to Fig. 2, it will be seen that the catheter tube 18 is formed with a lumen 20 that extends the length of the catheter tube 18. Further, Fig. 2 indicates that a deflection control wire 22 extends through the lumen 20 from an extracorporeal control mechanism 24. In particular, the control mechanism 24 includes a pivot arm 26 which can be rotated about the pivot point 28 by an operator (not shown) to exert a proximally directed force on the deflection control wire 22, and can include a brake or some other mechanism to maintain the deflection control wire 22 at a constant tension. It will be appreciated by the skilled artisan that the control mechanism 24 shown in Fig. 2 is only exemplary and that any device known in the pertinent art for generating an axial force on the deflection control wire 22 is suitable for the present invention. As best seen in Fig. 3, the deflection control wire 22 extends through the articulation segment 16 and attaches to the tip member 14.

Referring back to Fig. 2, the catheter 10 is shown to include a refrigerant source 30 which is to be used for the purpose of supplying a fluid that can be cooled to a temperature of approximately minus eighty degrees Celsius to the tip member 14. In a particular embodiment of the present invention, a medical gas, such as nitrous oxide, is used as the refrigerant. With cross reference to Figs. 2 and 3 it can be seen that the catheter 10 also includes a tube 32 that extends from the refrigerant source 30 and through the lumen 20 of the catheter tube 18 to the articulation segment 16. As further shown, tube 32 includes a feed line 34 to deliver refrigerant from the refrigerant source 30 to the articulation segment 16 and a return line 36 to deliver refrigerant back to the refrigerant source 30 from the articulation segment 16.

Referring now to Fig. 3. is can be seen that the articulation segment 16 of length, L, has a wall 38 that is formed with a first section 40 made of a first material having flexural modulus, M₁, and a second section 42 made of a second material having flexural modulus, M₂, with M₂ being larger than M₁ (M₂ > M₁). A preferred first material for the first section 40 is a polyether block amide (PEBA) such as a PEBAX® having a flexural modulus of approximately 0.2GPa. A preferred second material for the second section 42 is a polyamide such as "Nylon 12" having a flexural modulus of approximately 1.0GPa. As will be appreciated by the skilled artisan, several thermoplastic polyurethanes and elastomeric polyesters may be used. For the purposes of the present disclosure, the flexural modulus of anisotropic materials is measured in the direction of tube elongation. More specifically, as shown in Fig. 4, flexural modulus of anisotropic materials is determined by placing test samples in the test fixture 44 and oriented the sample so that sample direction 46 corresponds to a direction on the articulation segment 16 that is parallel to the longitudinal axis 48 of the articulation segment 16.

In the particular embodiment of the present invention shown in Figs. 3 and 5, the second section 42 (i.e. the high modulus material, M₂) is embedded in the wall 38 of the articulation segment 16. More specifically, as shown, the cylindrically shaped wall 38 extends from a cylindrical inner surface 50 to a cylindrical outer surface 52. At and near the inner surface 50, the wall 38 is made of low modulus material, M₁. Also, at and near the outer surface 52, the wall 38 is made of low modulus material, M₁. Between the inner surface 50 and outer surface 52, the second section 42 of high modulus material, M₂ is embedded in the wall 38. The second section 42 of high modulus material, M₂ preferably extends from the distal end 54 to the proximal end 56 of the articulation segment 16 (as shown in Fig. 3), and extends around the longitudinal axis 48 through an azimuthal angle, α₁, of approximately forty-five degrees (45°), as shown in Fig. 5. Also in this embodiment, an open lumen 58 can be formed in the wall 38 between the inner surface 50 and outer surface 52. Preferably, if used, the open lumen 58 is positioned approximately one-hundred and eighty degrees (180°) around the longitudinal axis 48 from the second section 42, and extends around the longitudinal axis 48 through an azimuthal angle, α₂, of approximately forty-five degrees (45°), as shown. Impliedly, open lumen 58 may be absent.

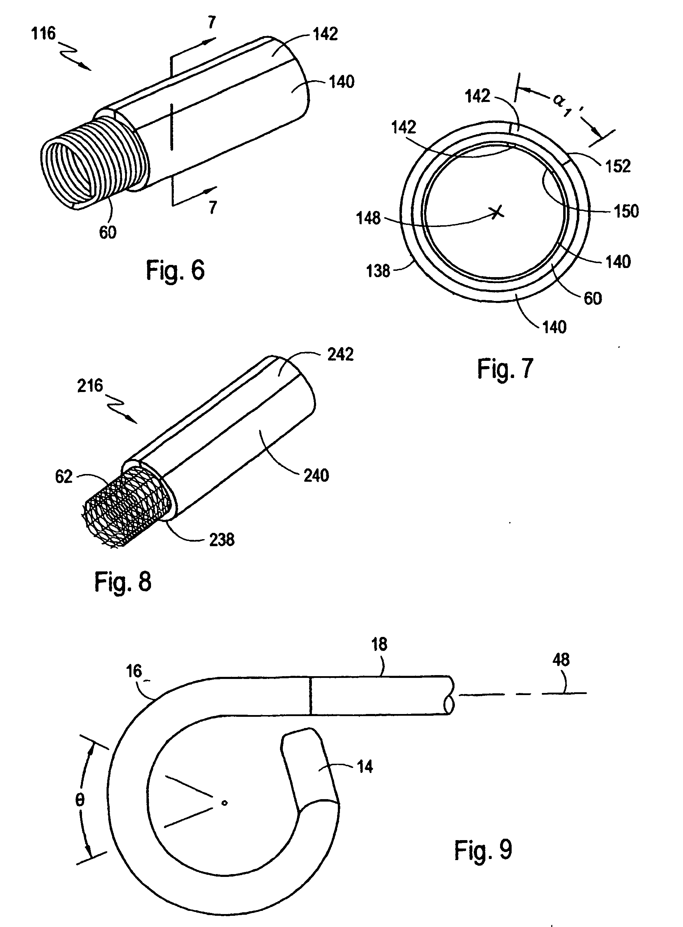

Figs. 6 and 7 show another particular embodiment of the articulation segment 116 having a wall 138 that is formed with a first section 140 made of a first material having flexural modulus, M₁, and a second section 142 made of a second material having flexural modulus, M₂, with M₂ being larger than M₁ (M₂ > M₁). In this embodiment, the second section 142 (i.e. the high modulus material, M₂) extends from the inner surface 150 of the wall 138 to the outer surface 152 of the wall 138. Like the embodiment described above, the second section 142 of high-modulus material, M₂ preferably extends the entire axial length of the articulation segment 116, and extends around the longitudinal axis 148 through an azimuthal angle, α₂', of approximately forty-five degrees (45°).

It can be further seen from Figs. 6 and 7 that a metallic coil 60 is embedded within the wall 138 between the inner surface 150 and outer surface 152, as shown. The metallic coil 60 is provided to axially stiffen the articulation segment 116, without significantly reducing the lateral flexibility of the articulation segment 116. Thus, the metallic coil 60 increases both the pushability and torqueability of the articulation segment 116 without significantly increasing the force necessary to deflect the distal end of the articulation segment 116 from the longitudinal axis 148.

Fig. 8 shows yet another particular embodiment of an articulation segment 216 formed with a first section 240 made of a first material having flexural modulus, M₁, and a second section 242 made of a second material having flexural modulus, M₂. with M₂ being larger than M₁ (M₂ > M₁). In this embodiment, a metallic braid 62 is embedded in the wall 238 of the articulation segment 216 to axially stiffen the articulation segment 216, without significantly reducing the lateral flexibility of the articulation segment 216.

With cross reference now to Figs. 2 and 9, it is to be appreciated that with the articulation segment 16 positioned within a patient's body, the control mechanism 24 can be selectively activated from an extracorporeal location to controllably deflect the tip member 14 and bend the articulation segment 16 through an angle, θ, that can be as large as approximately two-hundred seventy degrees (270°). It is to be further appreciated that the first and second sections 40, 42 are arranged relative to the deflection control wire 22 to ensure that the articulation segment 16 bends in a pre-selected bend plane in response to a movement of the deflection control wire 22. Selectively reconfiguring the shape of the articulation segment 16 in this manner can be performed to steer the catheter 10 through the vasculature of the body or to obtain a pre-selected shape for articulation segment 16 at the target tissue.

While the particular Catheter Having Articulation System as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for articulating a catheter (10) in the vasculature of a patient, said system comprising:
a co-extruded articulation segment (16,116,216) having an elongated, cylindrically shaped wall (38,138,238) extending from a distal end to a proximal end and surrounding a central lumen (20) therebetween, said wall defining a longitudinal axis (48,148) and formed with a first section (40,140,240) made of a first material having flexural modulus, M₁, and a second section (42,142,242) made of a second material having flexural modulus, M₂, wherein M₂ is larger than M₁ (M₂ > M₁), said second section of said articulation segment being elongated in a direction parallel to said longitudinal axis and subtending a substantially constant arc angle, α, of approximately forty-five degrees from said distal end to said proximal end;
a tip member (14) affixed to the distal end of said articulation segment;
a control wire (22) having a portion disposed in said central lumen of said articulation segment, said control wire having a first end and a second end with said first end attached to said tip member at an attachment point, said attachment point being distanced radially from said longitudinal axis; and
a means (24) engaged with the second end of said control wire for axially moving said control wire to selectively bend said articulation segment and deflect said tip member through an arc, θ, in a plane to articulate the catheter.

2. A system as recited in Claim 1 wherein said second section extends from said distal end of said wall to said proximal end of said wall.

3. A system as recited in Claim 1 wherein said first material is a polyether block amide.

4. A system as recited in Claim 1 wherein said second material is a polyamide.

5. A system as recited in Claim 1 wherein said attachment point, said longitudinal axis and a portion of said second section lie within a common plane.

6. A system as recited in Claim I wherein said arc θ has an arc length greater than approximately two hundred and seventy degrees (270°) during a deflection of said tip member.

7. A system as recited in Claim 1 wherein said wall has a length between said proximal end and said distal end and said length is greater than approximately ten millimeters.

8. A system as recited in Claim I further comprising a metallic coil spring (60) embedded in said wall to axially stiffen said articulation segment.

9. A system as recited in Claim 1 further comprising a metallic braid (62) embedded in said wall to axially stiffen said articulation segment.

10. A system as recited in Claim 1 wherein said wall is formed with an inner surface and an outer surface and wherein said second section extends from said inner surface to said outer surface.

11. A system as recited in Claim 1 wherein said wall is formed with an inner surface and an outer surface and wherein said second section is positioned between said inner surface and said outer surface and does not extend to said inner surface and does not extend to said outer surface, and wherein said wall is formed with an open lumen (58) positioned between said inner surface and said outer surface.

12. A system as recited in Claim 1 wherein said tip member is made of a thermally conductive material and said tip is in fluid communication with a cooling assembly which comprises:
a refrigerant source for providing a fluid having a temperature of approximately forty degrees Kelvin;
a tube extending through a proximal tube lumen and said articulation segment lumen to interconnect said refrigerant source in fluid communication with said tip member; and
a means for circulating said fluid through said tip member during a cardiac cryoablation procedure.

13. A system as recited in Claim 1 wherein said control wire is attached to said tip member at an attachment point positioned to interpose said longitudinal axis of said articulation segment between said attachment point and said second section.

14. A method for manufacturing a catheter having an articulation system, said method comprising the steps of:
co-extruding an elongated, cylindrically shaped wall (38,138,238) having a first section (40,140,240) made of a first material having flexural modulus, M₁, and a second section (42,142,242) made of a second material having flexural modulus, M₂, wherein M₂ is larger than M₁ (M₂ > M₁) and wherein said second section subtends a substantially constant arc angle, α, of approximately forty-five degrees from a distal end to a proximal end;
affixing a tip member (14) to said wall;
connecting a catheter tube to said wall;
attaching a control wire (22) to said tip member at an attachment point with the control wire extending proximally through said catheter tube; and
engaging said control wire with a control means (24) for pulling said control wire in a proximal direction to bend said wall and deflect said tip member through an arc, θ, in a plane defined by said second section of said wall and said attachment point.

15. A method recited in Claim 14 wherein said first material is a polyether block amide.

16. A method recited in Claim 14 wherein said second material is a polyamide.

17. A method as recited in Claim 14 wherein said wall extends from a distal end to a proximal end and wherein said second section extends from said distal end of said wall to said proximal end of said wall.

## Patentansprüche

1. System zum gelenkigen Anordnen eines Katheters (10) im Gefäßsystem eines Patienten, wobei das System aufweist:
ein koextrudiertes Gelenksegment (16, 116, 216) mit einer langgestreckten zylindrischen Wand (38, 138, 238), die sich von einem distalen Ende zu einem proximalen Ende erstreckt und ein zwischen diesen befindliches mittiges Lumen (20) umschließt, wobei die Wand eine Längsachse (48, 148) bildet und mit einem aus einem ersten Material mit einem Biegemodul M₁ gebildeten ersten Sektor (40, 140, 240) und einem aus einem zweiten Material mit einem Biegemodul M₂ gebildeten zweiten Sektor (42, 142, 242) ausgebildet ist, wobei M₂ größer als M₁ ist (M₂ > M₁), wobei der zweite Sektor des Gelenksegments in einer Richtung parallel zu der Längsachse langgestreckt ist und einen im wesentlichen konstanten Bogenwinkel α von ungefähr 45° von dem distalen Ende zu dem proximalen Ende einnimmt;
ein Spitzenteil (14), das an dem distalen Ende des Gelenksegments angebracht ist;
einen Steuerdraht (22), der einen in dem mittigen Lumen des Gelenksegments angeordneten Bereich aufweist, wobei der Steuerdraht ein erstes Ende und ein zweites Ende hat, wobei das erste Ende an einem Befestigungspunkt an dem Spitzenteil angebracht ist, wobei der Befestigungspunkt radial von der Längsachse beabstandet ist; und
eine mit dem zweiten Ende des Steuerdrahts zusammengreifende Einrichtung (24) zum axialen Bewegen des Steuerdrahtes, um zum gelenkigenBewegen des Katheters das Gelenksegment selektiv zu biegen und das Spitzenteil über einen Bogen θ in einer Ebene auszulenken.

2. System nach Anspruch 1, bei dem sich der zweite Sektor von dem distalen Ende der Wand zu dem proximalen Ende der Wand erstreckt.

3. System nach Anspruch 1, bei dem das erste Material ein Polyetherblockamid ist.

4. System nach Anspruch 1, bei dem das zweite Material ein Polyamid ist.

5. System nach Anspruch 1, bei dem der Befestigungspunkt, die Längsachse und ein Bereich des zweiten Sektors in einer gemeinsamen Ebene liegen.

6. System nach Anspruch 1, bei dem der Bogen θ während des Auslenkens des Spitzenteils eine Bogenlänge hat, die größer als ungefähr zweihundertsiebzig Grad (270°) ist.

7. System nach Anspruch 1, bei dem die Wand eine Länge zwischen dem proximalen Ende und dem distalen Ende hat und diese Länge größer als ungefähr 10 Millimeter ist.

8. System nach Anspruch 1, ferner mit einer Schraubenfeder (60) aus Metall, die in der Wand eingebettet ist, um das Gelenksegment zu versteifen.

9. System nach Anspruch 1, ferner mit einem Metallgeflecht (62), das in der Wand eingebettet ist, um das Gelenksegment zu versteifen.

10. System nach Anspruch 1, bei dem die Wand mit einer Innenfläche und einer Außenfläche ausgebildet ist und der zweite Sektor sich von der Innenfläche zu der Außenfläche erstreckt.

11. System nach Anspruch 1, bei dem die Wand mit einer Innenfläche und einer Außenfläche ausgebildet ist und der zweite Sektor zwischen der Innenfläche und der Außenfläche angeordnet ist und sich nicht zu der Innenfläche und nicht zu der Außenfläche erstreckt, und wobei die Wand mit einem offenen Lumen (58) ausgebildet ist, das zwischen der Innenfläche und der Außenfläche angeordnet ist.

12. System nach Anspruch 1, bei dem das Spitzenteil aus einem wärmeleitfähigen Material besteht und die Spitze in Fluidverbindung mit einer Kühlanordnung steht, welche aufweist:
eine Kältemittelquelle zum Zuführen eines Fluids mit einer Temperatur von ungefähr vierzig Grad Kelvin;
ein Rohr, das sich durch das Lumen eines proximalen Rohres und das Lumen des Gelenksegments erstreckt, um eine Fluidverbindung zwischen der Kältemittelquelle und dem Spitzenteil herzustellen; und
eine Einrichtung zum Zirkulierenlassen des Fluids durch das Spitzenteil während eines Kryoablationsvorgangs am Herzen.

13. System nach Anspruch 1, bei dem der Steuerdraht an dem Spitzenteil an einem Befestigungspunkt angebracht ist, welcher derart positioniert ist, dass die Längsachse des Gelenksegments zwischen dem Befestigungspunkt und dem zweiten Sektor angeordnet ist.

14. Verfahren zur Herstellung eines Katheters mit einem Gelenksystem, wobei das Verfahren die folgenden Schritte aufweist:
Koextrudieren einer langgestreckten, zylindrischen Wand (38, 138, 238) mit einem ersten Sektor (40, 140, 240), der aus einem ersten Material mit einem Biegemodul M₁ besteht, und einem zweiten Sektor (42, 142, 242), der aus einem zweiten Material mit einem Biegemodus M₂ besteht, wobei M₂ größer als M₁ ist (M₂ > M₁), und wobei der zweite Sektor einen im wesentlichen konstanten Bogenwinkel α von ungefähr 45° von einem distalen Ende zu einem proximalen Ende einnimmt;
Befestigen eines Spitzenteils (14) an der Wand;
Verbinden eines Katheterrohrs mit der Wand;
Anbringen eines Steuerdrahts (22) an dem Spitzenteil an einem Befestigungspunkt, wobei sich der Steuerdraht proximal durch das Katheterrohr erstreckt; und
Anschließen des Steuerdrahts an eine Steuereinrichtung (24) zum Ziehen des Steuerdrahts in eine proximale Richtung, um die Wand zu biegen und das Spitzenteil über einen Bogen θ in einer Ebene auszulenken, welche durch den zweiten Sektor der Wand und den Befestigungspunkt definiert ist.

15. Verfahren nach Anspruch 14, bei dem das erste Material ein Polyetherblockamid ist.

16. Verfahren nach Anspruch 14, bei dem das zweite Material ein Polyamid ist.

17. Verfahren nach Anspruch 14, bei dem sich die Wand von einem distalen Ende zu einem proximalen Ende erstreckt und sich der zweite Sektor von dem distalen Ende der Wand zu dem proximalen Ende der Wand erstreckt.

## Revendications

1. Système pour articuler un cathéter (10) dans la vasculature d'un patient, ledit système comprenant :
un segment d'articulation coextrudé (16, 116, 216) comportant une paroi de forme cylindrique allongée (38, 138, 238) s'étendant à partir d'une extrémité distale vers une extrémité proximale et entourant une lumière centrale (20) entre celles-ci, ladite paroi définissant un axe longitudinal (48, 148) et étant formée avec une première partie (40, 140, 240) faite d'un premier matériau ayant un module d'élasticité en flexion, M₁, et une seconde partie (42, 142, 242) faite d'un second matériau ayant un module d'élasticité en flexion, M₂, M₂ étant plus grand que M₁ (M₂ > M₁), ladite seconde partie dudit segment d'articulation étant allongée dans une direction parallèle audit axe longitudinal et sous-tendant un angle à arc sensiblement constant, α, d'approximativement quarante-cinq degrés à partir de ladite extrémité distale vers ladite extrémité proximale ;
un élément de pointe (14) fixé à l'extrémité distale dudit segment d'articulation ;
un fil de commande (22) comportant une partie disposée dans ladite lumière centrale dudit segment d'articulation, ledit fil de commande comportant une première extrémité et une seconde extrémité, ladite première extrémité étant attachée audit élément de pointe en un point de fixation, ledit point de fixation étant éloigné de façon radiale dudit axe longitudinal ; et
des moyens (24) en prise avec la seconde extrémité dudit fil de commande pour déplacer de façon axiale ledit fil de commande afin de plier de façon sélective ledit segment d'articulation et fléchir ledit élément de pointe par un arc, θ, dans un plan afin d'articuler le cathéter.

2. Système selon la revendication 1, dans lequel ladite seconde partie s'étend depuis ladite extrémité distale de ladite paroi vers ladite extrémité proximale de ladite paroi.

3. Système selon la revendication 1, dans lequel ledit premier matériau est un amide séquencé de polyéther.

4. Système selon la revendication 1, dans lequel ledit second matériau est un polyamide.

5. Système selon la revendication 1, dans lequel ledit point de fixation, ledit axe longitudinal et une partie de ladite seconde partie se trouvent dans un plan commun.

6. Système selon la revendication 1, dans lequel ledit arc θ présente une longueur d'arc supérieure à approximativement deux cent soixante-dix degrés (270°) lors d'un fléchissement dudit élément de pointe.

7. Système selon la revendication 1, dans lequel ladite paroi présente une longueur comprise entre ladite extrémité proximale et ladite extrémité distale, et ladite longueur est supérieure à approximativement dix millimètres.

8. Système selon la revendication 1, comprenant en outre un ressort hélicoïdal métallique (60) inclus dans ladite paroi afin de rigidifier de façon axiale ledit segment d'articulation.

9. Système selon la revendication 1, comprenant en outre une tresse métallique (62) incluse dans ladite paroi afin de rigidifier de façon axiale ledit segment d'articulation.

10. Système selon la revendication 1, dans lequel ladite paroi est formée avec une surface interne et une surface externe et dans lequel ladite seconde partie s'étend depuis ladite surface interne vers ladite surface externe.

11. Système selon la revendication 1, dans lequel ladite paroi est formée avec une surface interne et une surface externe et dans lequel ladite seconde partie est positionnée entre ladite surface interne et ladite surface externe et ne s'étend pas vers ladite surface interne et ne s'étend pas vers ladite surface externe, et dans lequel ladite paroi est formée avec une lumière ouverte (58) positionnée entre ladite surface interne et ladite surface externe.

12. Système selon la revendication 1, dans lequel ledit élément de pointe est fait d'un matériau thermiquement conducteur et ladite pointe est en communication fluidique avec un ensemble de refroidissement qui comprend :
une source de réfrigérant destinée à fournir un fluide ayant une température d'approximativement quarante degrés Kelvin ;
un tube s'étendant à travers une lumière de tube proximale et ladite lumière du segment d'articulation afin d'interconnecter ladite source de frigorigène en communication fluidique avec ledit élément de pointe ; et
des moyens pour faire circuler ledit fluide à travers ledit élément de pointe lors d'une intervention de cryoablation cardiaque.

13. Système selon la revendication 1, dans lequel ledit fil de commande est attaché audit élément de pointe en un point de fixation positionné pour interposer ledit axe longitudinal dudit segment d'articulation entre ledit point de fixation et ladite seconde partie.

14. Procédé pour fabriquer un cathéter comportant un système d'articulation, ledit procédé comprenant les étapes consistant à :
co-extruder une paroi de forme cylindrique allongée (38, 138, 238) comportant une première partie (40, 140, 240), faite d'un premier matériau ayant un module d'élasticité en flexion, M₁, et d'une seconde partie (42, 142, 242) faite d'un second matériau ayant un module d'élasticité en flexion, M₂, M₂ étant plus grand que M₁ (M₂ > M₁) et dans lequel ladite seconde partie sous-tend un angle à arc sensiblement constant, α, d'approximativement quarante-cinq degrés à partir d'une extrémité distale vers une extrémité proximale ;
fixer un élément de pointe (14) à ladite paroi ;
connecter un tube de cathéter à ladite paroi ;
fixer un fil de commande (22) audit élément de pointe en un point de fixation, le fil de commande s'étendant de façon proximale à travers ledit tube de cathéter ; et
mettre en prise ledit fil de commande avec des moyens de commande (24) destinés à tirer ledit fil de commande dans une direction proximale afin de plier ladite paroi et fléchir ledit élément de pointe par un arc, θ, dans un plan défini par ladite seconde partie de ladite paroi et ledit point de fixation.

15. Procédé selon la revendication 14, dans lequel ledit premier matériau est un amide séquencé de polyéther.

16. Procédé selon la revendication 14, dans lequel ledit second matériau est un polyamide.

17. Procédé selon la revendication 14, dans lequel ladite paroi s'étend à partir d'une extrémité distale vers une extrémité proximale et dans lequel ladite seconde partie s'étend à partir de ladite extrémité distale de ladite paroi vers ladite extrémité proximale de ladite paroi.
